Europäisches Patentamt

European Patent Office

Office européen des brevets

(11) Publication number: **0 188 567**
**B1**

(12) **EUROPEAN PATENT SPECIFICATION**

(45) Date of publication of patent specification: **24.01.90**

(51) Int. Cl.⁵: **F 04 D 29/12**

(21) Application number: **85903734.3**

(22) Date of filing: **05.07.85**

(86) International application number:
**PCT/US85/01264**

(87) International publication number:
**WO 86/00672 30.01.86 Gazette 86/03**

(54) **CENTRIFUGAL BLOOD PUMP WITH TAPERED SHAFT SEAL.**

(30) Priority: **09.07.84 US 628727**
**09.07.84 US 628756**

(43) Date of publication of application:
**30.07.86 Bulletin 86/31**

(45) Publication of the grant of the patent:
**24.01.90 Bulletin 90/04**

(84) Designated Contracting States:
**AT BE CH DE FR GB IT LI LU NL SE**

(56) References cited:
**GB-A- 669 881**
**SE-A- 159 371**
**US-A- 865 128**
**US-A-2 106 884**
**US-A-2 154 199**
**US-A-2 157 597**
**US-A-2 207 371**
**US-A-2 243 227**
**US-A-3 366 068**
**US-A-3 430 921**
**US-A-3 604 098**
**US-A-4 380 416**

(73) Proprietor: **MINNESOTA MINING AND MANUFACTURING COMPANY**
**3M Center, P.O. Box 33427**
**St. Paul, Minnesota 55133 (US)**

(72) Inventor: **CLAUSEN, Earl, W.**
**3121 South Highway 101**
**Wayzata, MN 55391 (US)**
Inventor: **HUBBARD, Lloyd, C.**
**4818 Ridge Road**
**Minnetonka, MN 55343 (US)**

(74) Representative: **Fry, Alan Valentine**
**FRY HEATH & CO. Seloduct House Station Road**
**Redhill Surrey RH1 1NF (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is related to centrifugal blood pumps.

Centrifugal pumps have been used for many years to pump a wide variety of different fluid materials. In general, a centrifugal pump includes a pumping chamber with an inlet, an outlet, a drive shaft, and an impeller attached to the drive shaft for rotation within the chamber. As the impeller is rotated, it imparts centrifugal force to the fluid, thus pumping the fluid from the pump inlet to the pump outlet.

In recent years, centrifugal pumps have been used extensively for pumping blood during open heart surgery. Examples of centrifugal blood pumps are shown in the following U.S. patents: Rafferty et al Re. 28,742; Dorman et al 3,608,088; Rafferty et al 3,647,324; Kletschka et al 3,864,055; Rafferty et al 3,957,389; Rafferty et al 3,970,408; Rafferty et al 4,037,984; and Reich et al 4,135,253.

The pumping of blood requires great care to avoid any damage to the red corpuscles, or any of the other constituents of blood. Any practical blood pump useful as part of heart/lung bypass equipment during open heart surgery must deliver the requisite flow volumes under pressure, without damaging the blood being pumped.

In a centrifugal pump, and in particular in a centrifugal pump for pumping liquids such as blood, a fluid tight seal between the drive shaft and the housing is an important factor in the performance of the pump. Friction at the seal produces heat which can damage both the components of the pump and the blood being pumped if not dissipated.

In prior art centrifugal pumps, the rotation of the impeller can lead to generation of an air bubble surrounding the shaft. This air bubble tends to seek the smallest shaft diameter, which is adjacent the drive shaft seal. The air bubble tends to insulate the seal from the flow of the fluid within the pump chamber, thus decreasing the dissipation of heat generated by friction at the seal interface.

The present invention is an improved centrifugal blood pump which has a tapered seal between the pump housing wall and the hub of the impeller for providing a fluid tight seal interface which surrounds the drive shaft at an intermediate position between the wall and the hub. This tapered seal has a maximum radial dimension adjacent the wall and a minimum radial dimension adjacent the hub. The hub has a generally conical shape with a maximum diameter adjacent the tapered seal.

With the pump of the present invention, the seal is tapered so that air bubbles (which seek the smallest shaft diameter) will not insulate the seal interface edges from fluid flow. In addition, the seal interface is located in a high flow area between the wall and the hub to increase cooling.

In preferred embodiments, the tapered seal includes a seal stator which is connected to the wall, a seal rotor which is rotatable with and movable axially on the drive shaft, and a resilient ring positioned between the hub and the seal rotor. The seal stator surrounds the drive shaft and defines a first seal face which is generally transverse to the axis and which is located at an intermediate position between the wall and the hub. The seal rotor is positioned coaxially on the drive shaft and defines a second seal face which faces the first seal face and which is generally transverse to the axis. The resilient ring urges the second seal face into engagement with the first seal face to provide the fluid tight seal interface around the drive shaft.

The invention will now be described by way of example only with reference to the accompanying diagrammatic drawings in which:

Figure 1 is a front view of the centrifugal pump of the present invention.

Figure 2 is a side view of the centrifugal pump.

Figure 3 is a sectional view of the centrifugal pump along section 3—3 of Figure 1.

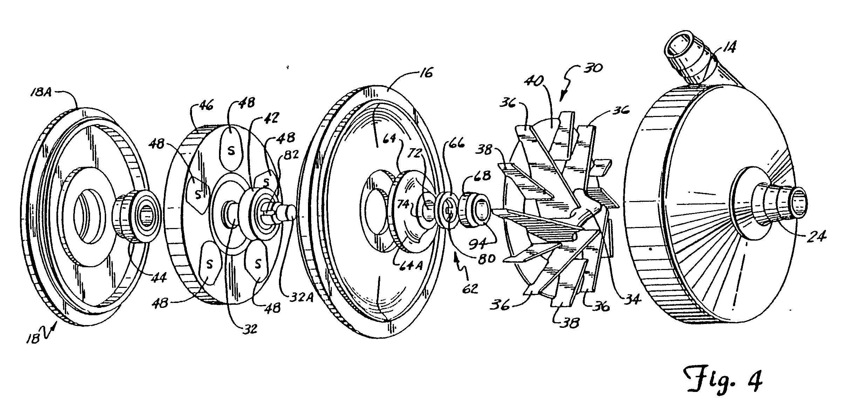

Figure 4 is an exploded perspective view of the centrifugal pump.

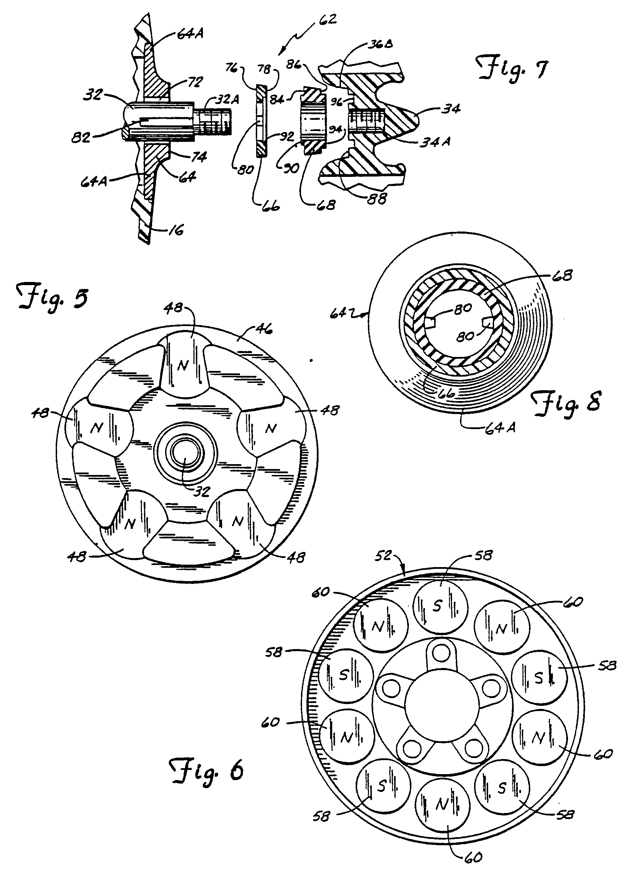

Figure 5 is a view of the rotor along view 5—5 of Figure 3.

Figure 6 is a view of the drive plate along view 6—6 of Figure 3.

Figure 7 is an exploded view, partially in section, of the tapered shaft seal of the centrifugal pump.

Figure 8 is a sectional view of the shaft seal along section 8—8 of Figure 3.

In the preferred embodiment shown in the Figures, centrifugal pump 10 of the present invention includes a three-part housing 12 formed by front housing section 14, centre wall housing section 16, and rear housing section 18. Front and centre sections 14 and 16 are sealed to define pumping chamber 20. Centre and rear sections 16 and 18 are sealed to define rotor chamber 22.

Front housing section 14 (which is preferably transparent so that operation of the pump can be visually monitored) includes axially aligned pump inlet 24 and tangential pump outlet 26. Blood or other biological fluid is received at inlet 24 from inlet tubing 27 and is pumped to outlet 26 and outlet tubing 28 by rotation of impeller 30 within pumping chamber 20.

Impeller 30 is mounted on a threaded outer end 32A of shaft 32, and is rotated about an axis defined by shaft 32. Impeller 30 includes a conical shaped impeller hub 34 (with internal threads 34A for engaging threaded outer end 32A), a plurality of long blades 36, a plurality of short blades 38, and circular flange 40.

Long blades 36 are attached at their inner ends to impeller hub 38. Flange 40 is attached to and is supported by the lower edges of long blades 36. Short blades are supported by flange 40. In the particular embodiments shown in the Figures, long and short blades 36 and 38 are alternatively spaced about the circumference of impeller 30.

Large diameter impellers require a greater number of blades in order to achieve pumping efficiency. By use of short blades 38 supported by flange 40, impeller 30 achieves pumping efficiency while retaining a small hub diameter, since only long blades 36 are attached to hub 34.

Shaft 32 is mounted for rotation by a pair of axially aligned ball bearing 42 and 44. Ball bearing 42 is press fitted into centre wall section 16, while ball bearing 44 is press fitted into rear housing section 18.

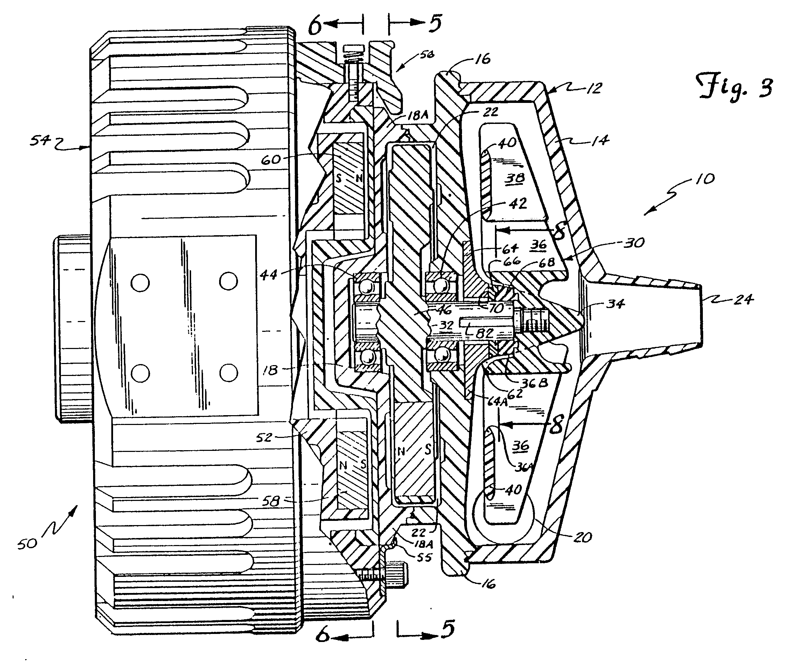

Rotor 46 is connected to shaft 32, so that as rotor 46 rotates within rotor chamber 22, shaft 32 and impeller 30 are rotated. In the particular embodiment shown in the Figures, pump 10 is a magnetically driven pump. Rotor 46 carries a plurality of small ceramic disk magnets 48. Each magnet 48 has the same pole orientation (which is the particular embodiment shown) has the north (N) pole closest to drive console 50. Magnets 48 are equally spaced around the circumference of rotor 46 and, in the particular embodiment shown in Figure 3, five magnets 48 spaced at 72° intervals from one another are carried by rotor 46.

Drive console 50 includes drive plate 52 which is rotated by motor 54 about an axis which is aligned with the axis of shaft 32. Clip 55 and spring-loaded latch 56 engage flange 18A of rear housing section 18 to hold pump housing 12 in position adjacent drive console 50. Pump housing 12 can be quickly removed from engagement with drive console 50 by lifting latch 56.

Drive plate 52 carries five equally spaced south (S) pole ceramic disk magnets 58 and five equally spaced ceramic disk north (N) pole magnets 60. Magnets 58 and 60 are arranged alternately (as shown in Figure 6). This gives both attractive and repelling force to magnets 48 carried by rotor 46. This magnetic drive allows the use of small, discrete magnets in pump 10, rather than a single large magnet with multiple poles. This provides a significant cost reduction which is of particular advantage since pump housing 12, when used for pumping blood or other biological fluids, must be disposed of after a single use.

In the present invention, leakage of fluid from pumping chamber 20 into rotor chamber 22 is prevented by a tapered shaft seal 62 formed by seal stator 64, seal rotor 66, and resilient elastomer spring 68. Tapered seal 62 is tapered to conform to the taper of impeller hub 34 so that an air bubble (which seeks the smallest shaft diameter within pumping chamber 20) will not insulate the seal interface edges from fluid flow. Tapered seal 62 provides a seal interface 70 between seal stator 64 and seal rotor 66 which is generally perpendicular to the axis of shaft 32 and which is located at an intermediate position between wall 16 and hub 34. The location of the seal interface 70 is in a high fluid flow area, which increases cooling effects and improves dissipation of heat caused by friction at seal interface 70.

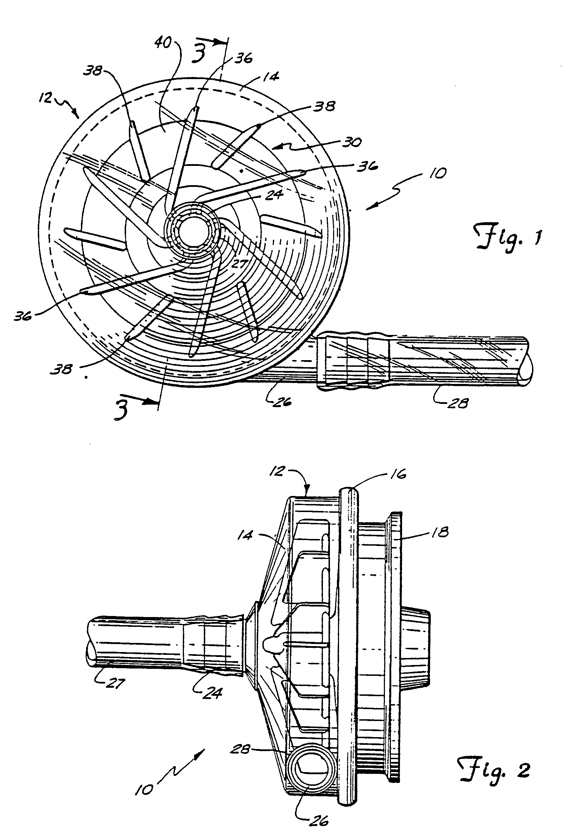

In the preferred embodiment of the present invention shown in the drawings seal stator 64 is fixed to wall 16. Seal stator 64 is a high thermal conductivity material (preferably nickel-plated aluminium). Seal stator 64 has a central passage 72 which is axially aligned with shaft 32 and is of sufficient diameter so that shaft 32 does not contact seal stator 64. Front face 74 of seal stator 64 defines the location of seal interface 70 and is, in the preferred embodiment shown in Figures 1 and 2, generally perpendicular to the axle of shaft 32.

Seal stator 64 has a flange 64A at its rear end which extends outward in a radial direction and generally conforms to the surface of wall 16 at the rear end of pumping chamber 20. Flange 64A provides a large surface area for seal stator 64, thus increasing the ability of seal stator 64 to transfer heat generated at seal interface 70.

Seal stator 66 is positioned on shaft 32 adjacent to seal stator 64. Rear face 76 of seal rotor 66 engages the front face of seal stator 64 to provide seal interface 70. Front face 78 of seal rotor 66 faces and is engaged by spring 68. Seal rotor 66 has a pair of inwardly projecting keys 80 which engage axially extending keyways 82 on shaft 32 so that seal rotor 66 can move in the axial direction and yet rotates with shaft 32. In a preferred embodiment, seal rotor 66 is a low friction polymer material such as nylon.

Spring 68 is an elastomer (such as silicone rubber) ring which is mounted coaxially on shaft 32 between impeller hub 34 and seal rotor 66. Rear face 84 of spring 68 engages front face 78 of seal rotor 66, and front face 86 of spring 68 engages rear face 88 or hub 34. Elastomer spring 68 is maintained under compression by hub 34, which is threaded on outer end 32A of shaft 32, so that it urges seal rotor 66 in an axial direction into engagement with seal stator 64. Spring 68 preferably has an annular rib 90 which is positioned in annular groove 92 in front face 78 of seal rotor 66 and has an annular rib 94 which is positioned in annular groove 96 in the rear face 98 of hub 34. Ribs 90 and 94 help to maintain an axial alignment of spring 68 so that an essentially uniform axial force is applied to seal rotor 66.

To increase fluid flow in the area of seal interface 70, each of the long blades 36 of impeller 30 has a lower edge 36A which is below the lower edge of impeller hub 34. Each long blade 36 has an inner edge 36B which extends from lower edge 36A to impeller hub 34, and which is closely spaced and generally parallel to the outer surface of tapered seal 62.

Although the present invention has been described with reference to preferred embodiments, workers skilled in the art will recognize that changes may be made in form and detail without departing from the spirit and scope of the invention. For example, although the particular embodiment of pump 10 shown in the Figures utilises a synchronous magnetic drive, the shaft seal is equally applicable to pumps in which other forms of coupling (including direct coupling) between shaft 32 of pump 10 and motor 54 of console 50 are provided.

As a further example, although as shown in the Figures flange 40 is attached to long blades 36 near their rear edges, in other embodiments flange 40 is connected to long blades 36 at other points such as their front edges or a location between the front and rear edges.

## Claims

1. A centrifugal pump (10) for pumping a biological fluid such as blood, the pump comprising a pump housing (12) having a pumping chamber (20) therein and having an inlet (24) and an outlet (26) connected to the pumping chamber (20); a shaft (32) extending in an axial direction into the pump housing (12) to define a rotational axis; an impeller (30) for rotation about the rotational axis within the pumping chamber (20), the impeller (30) having a hub (34); the pump being characterized in that a tapered seal (62) is positioned between a wall (16) of the pump housing (12) and the hub (34) to provide a fluid-tight seal interface (70) surrounding the shaft (32) at an intermediate position between the wall (16) and the hub (34), the tapered seal (62) having a maximum radial dimension adjacent the wall (16) and a minimum radial dimension adjacent the hub (34); another surface of the tapered seal (61) and an outer surface of the hub (34) forming an essentially continuous tapered outer surface so that the seal interface (70) is located in a high fluid flow area within the housing (12) and so that trapped air within the biological fluid will not tend to accumulate adjacent the seal interface (70); and the hub (34) having a generally conical shape with a maximum diameter adjacent the tapered seal (62).

2. A centrifugal pump as claimed in Claim 1 characterised in that the seal interface (70) is generally transverse to the axial direction.

3. A centrifugal pump as claimed in Claim 2 characterised in that the tapered seal comprises a seal stator (64) connected to the wall (16) and surrounding the shaft (32), the seal stator (64) defining a first seal face (74) which is generally transverse to the axial direction and is located intermediate the wall (16) and the hub (34); a seal rotor (66) positioned coaxially on the shaft (32) between the seal stator (64) and the hub (34), the seal rotor (66) defining a second seal face (76) which faces the first seal face (74) and which is generally transverse to the axial direction, the seal rotor (66) being mounted for rotation with the impeller (30) and being movable in the axial direction; and a resilient ring (68) positioned coaxially on the shaft (32) for urging the first and second seal faces (74, 76) into engagement to provide the fluid-tight seal interface (70) around the shaft (32).

4. A centrifugal pump as claimed in Claim 3 characterised in that the seal stator (64) has a radially outwardly extending flange (64A) at an end closest to the wall (16).

5. A centrifugal pump as claimed in Claim 3 characterised in that the seal stator (64) is constructed from a metal; the seal rotor (66) is constructed from a low friction polymer ring; and the resilient ring (68) is constructed from an elastomer spring ring.

6. A centrifugal pump as claimed in Claim 5 characterised in that the seal stator (64) is constructed from aluminium; the low friction polymer is a nylon material; and the elastomer is a silicone rubber.

7. A centrifugal pump as claimed in Claim 3 characterised in that the resilient ring (68) is positioned between the hub (34) and the seal rotor (66) to urge the seal rotor (66) towards the seal stator (64).

8. A centrifugal pump as claimed in Claim 7 characterised in that the seal rotor (66) has an annular groove (92) in a surface facing the resilient ring (68), and in that the resilient ring (68) has an annular rib (90) which engages the annular groove (92) in the seal rotor (66).

9. A centrifugal pump as claimed in Claim 7 characterised in that the hub (34) has an annular groove (96) in a surface (88) facing the resilient ring (68), and in that the resilient ring (68) has an annular rib (94) which engages the annular groove (96) in the hub (34).

10. A centrifugal pump as claimed in any one of Claims 1 to 9 characterised in that the shaft (32) is a drive shaft which extends through the wall (16) with a first end (32A) within the pump chamber (20) and a second end on an opposite side of the wall (16) from the pumping chamber (20); and in that the hub (34) is attached to the first end (32A) of the drive shaft.

11. A centrifugal pump as claimed in any one of Claims 1 to 10 characterised in that the impeller (30) has a plurality of first blades (36) attached to and extending outward from the hub (34), each of the first blades (36) having a rear edge (36A) which is generally parallel to the wall (16) and which is positioned closer to the wall (16) than is the hub (34) and each having an inner edge (36B) which extends from the rear edge of the hub (34) and which is generally parallel to and closely spaced from an outer surface of the seal (62).

12. A centrifugal pump as claimed in Claim 11 characterised in that the rear edges (36A) of the first blades (36) are more closely spaced to the wall (16) than is the seal interface (70).

13. A centrifugal pump as claimed in Claim 11 characterised in that the impeller (30) further includes a blade support ring (40) supported by the plurality of first blades (36) at a position which is spaced radially outward from the hub (34) and the seal (62), and a plurality of a second, shorter blades (38) supported by the blade support ring (40) at positions between the first blades (36), and the second blades (38) being spaced radially from the hub (34).

14. A centrifugal pump as claimed in Claim 13 characterised in that the blade support ring (40) has an inner edge which is spaced radially from the hub (34), and wherein the first and second blades (36, 38) extend outward beyond an outer edge of a blade support ring (40).

**Patentansprüche**

1. Zentrifugalpumpe (10) zum Pumpen einer biologischen Flüssigkeit, wie Blut; die Pumpe hat ein Pumpengehäuse (12) mit einer Pumpkammer (20) darin und die Pumpe hat einen Einlaß (24) und einen Auslaß (26), die an die Pumpenkammer (20) angeschlossen sind; die Pumpe hat eine Welle (32), die sich in axialer Richtung in das Pumpengehäuse (12) erstreckt und eine Rotationsachse bildet; die Pumpe hat einen Propeller (30), der un die Rotationsachse innerhalb der Pumpkammer (30) drehbar ist und der eine Nabe (34) hat: die Pumpe ist dadurch gekennzeichnet, daß eine verjüngt gestaltete Dichtung (62) zwischen einer Wand (16) des Pumpengehäuse (12) und der Nabe (34) angeordnet ist, um eine flüssigkeitsadichte Dichtungsschnittstelle (70) zu beiten, die die Welle (32) in einer Zwischenposition zwischen der Wand (16) und der Nabe (34) umgibt, daß die verjüngte Dichtung (62) eine größte radiale Ausdehnung benachbart der Wand (16) hat und eine geringste radiale Ausdehnung benachbart zur Nabe (34) hat, daß eine weitere Oberfläche der verjüngten Dichtung (61) und eine Außenoberfläche der Nabe (34) eine eigentümlich fortlaufend verjüngte äußere Oberfläche bilden, so daß die Dichtungsschnittstelle (70) in einem Bereich einer starken Flüssigkeitsströmung innerhalb des Gehäuses (12) angeordnet ist und so daß in der biologischen Flüssigkeit gefangene Luft nicht bestrebt ist, sich neben der Dichtungsschnittstelle (70) zu sammeln, und daß die Nabe (34) eine im wesentlichen konische Gestalt hat, die einen größten Durchmesser anschließend zu der verjüngten Dichtung (62) hat.

2. Zentrifugalpumpe nach Anspruch 1, dadurch gekennzeichnet, daß die Dichtungsschnittstelle (70) im wesentlichen quer zur axialen Richtung liegt.

3. Zentrifugalpumpe nach Anspruch 2, dadurch gekennzeichnet, daß die verjüngte Dichtung einen Dichtungsstator (4) beinhaltet, der mit der Wand (16) verbunden ist und die Welle (32) umgibt sowie eine erste Dichtungsfläche (74) bestimmt, die im wesentlichen quer zur axialen Richtung liegt und zwischen der Wand (16) und der Nabe (34) angeordnet ist, daß ein Dichtungsrotor (66) koaxial auf der Welle (32) zwischen dem Dichtungsstator (64) und der Nabe (34) angeordnet ist und der Dichtungsrotor (66) eine zweite Dichtungsfläche (76) bestimmt, die gegen die erste Dichtungsfläche (74) gerichtet ist und im wesentlichen quer zur axialen Richtung liegt, daß der den Dichtungsrotor (66) mit dem Propeller (30) drehbar montiert ist und in der axialen Richtung verschieblich ist und daß ein elastischer Ring (68) koaxial auf der Welle (32) angeordnet ist um die erste und zweite Dichtungsfläche (74, 76) zusammenzudrücken um eine flüssigkeitsdichte Dichtungsschnittstelle (70) um die Welle (32) zu bilden.

4. Zentrifugalpumpe nach Anspruch 3, dadurch gekennzeichnet, daß der Dichtungsstator (64) einen sich radial nach außen erstreckenden Flansch (64A) an einem Ende nächst zu der Wand (16) hat.

5. Zentrifugalpumpe nach Anspruch 3, dadurch gekennzeichnet, daß der Dichtungsstator (64) aus Metall hergestellt ist, daß der Dichtungsrotor (66) aus einem hochgleitfähigem Ring aus einem Polymer hergestellt ist und daß der elastische Ring (68) aus einem Federring aus einem Elastomer hergestellt ist.

6. Zentrifugalpumpe nach Anspruch 5, dadurch gekennzeichnet, daß der Dichtungsstator (64) aus Aluminium hergestellt ist, daß das hochgleitfähige Polymer ein Polyamid ist und daß das Elastomer ein Silikonkautschuk ist.

7. Zentrifugalpumpe nach Anspruch 3, dadurch gekennzeichnet, daß der elastische Ring (68) zwischen der Nabe (34) und dem Dichtungsrotor (66) angeordnet ist, um den Dichtungsrotor (66) gegen den Dichtungsstator (64) zu drücken.

8. Zentrifugalpumpe nach Anspruch 7, dadurch gekennzeichnet, daß der Dichtungsrotor (66) eine umlaufende Nut (92) in einer Oberfläche hat, die gegen den elastischen Ring (68) gerichtet ist, und daß der elastische Ring (68) eine umlaufende Rippe (90) trägt, die in die umlaufende Nut (92) in dem Dichtungsrotor (66) eingreift.

9. Zentrifugalpumpe nach Anspruch 7, dadurch gekennzeichnet, daß die Nabe (34) eine umlaufende Nut (96) in einer Oberfläche (88) hat, die gegen den elastischen Ring (68) gerichtet ist, und daß der elastische Ring (68) eine umlaufende Rippe (94) trägt, der in die umlaufende Nut (96) in der Nabe (34) eingreift.

10. Zentrifugalpumpe nach einem der Ansprüche 1 bis 9, dadurch gekennzeichnet, daß die Welle (32) eine Antriebswelle ist, die sich durch die Wand (16) mit einem ersten Ende (32A) in die Pumpkammer (20) erstreckt und mit einem zweiten Ende in eine entgegengesetzte Seite der Wand (16) von der Pumpkammer (20) erstreckt, und daß die Nabe (34) an das erste Ende (32A) der Antriebswelle befestigt ist.

11. Zentrifugalpumpe nach einem der Ansprüche 1 bis 10, dadurch gekennzeichnet, daß der Propeller (30) eine erste Vielzahl von ersten Blättern (36) trägt, die sich von der Nabe (34) nach außen erstrecken, wobei jedes der ersten Blätter (36) eine Hinterkante (36A) hat, die im wesentlichen parallel zur Wand (16) liegt und die näher zur Wand (16) angeordnet ist als die Nabe (34), und wobei jedes eine Innenkante (36B) hat, die sich von der Hinterkante der Nabe (34) erstreckt und die im wesentlichen parallel zu und dicht benachbart von einer äußeren Oberfläche der Dichtung (62) ist.

12. Zentrifugalpumpe nach Anspruch 11, dadurch gekennzeichnet, daß die Hinterkanten (36A) der ersten Blätter (36) näher als die Dichtungsschnittstelle (70) zu der Wand (16) beabstandet sind.

13. Zentrifugalpumpe nach Anspruch 11, dadurch gekennzeichnet, daß die Propeller (30) einen Flügelstützring (40) enthält, der von der Vielzahl der ersten Blätter (36) in einer radial außerhalb von der Nabe (34) beabstandeten Lage gehalten ist, und daß eine Vielzahl von zweiten, kürzeren Blättern (38) in Lagen zwischen den ersten Blättern (36) durch den Flügelstützring (40) gehalten sind und daß die zweiten Blätter (38) radial von der Nabe (34) beabstandet sind.

14. Zentrifugalpumpe nach Anspruch 13, dadurch gekennzeichnet, daß der Flügelstützring (40) eine Innenkante hat, die radial von der Nabe (34) beabstandet ist, und daß die ersten und zweiten Blätter (36, 38) sich nach außen über eine Außenkante des Flügelstützringes (40) erstrecken.

## Revendications

1. Une pompe centrifuge (10) pour pomper un fluide biologique tel que le sang, ladite pompe se composant d'un carter (12) de pumpe comprenant une chambre d'aspiration (20) ainsi qu'une entrée (24) et une sortie (26) raccordées à la chambre d'aspiration (20); un arbre (32) disposé dans le sens axial et pénétrant dans la carter (12) de pompe afin de définir un axe de rotation; une roue mobile (30) tournant autour de l'axe de rotation à l'intérieur de la chambre d'aspiration (20), cette roue mobile (30) ayant un moyeu (34); la pompe étant caractérisé par le fait qu'on joint conique (62) est situé entre une paroi (16) du carter (12); de pompe et le moyeu (34) afin de fournir une interface (70) étanche aux fluides autour de l'arbre (32) à une position intermédiaire entre le paroi (16) et le moyeu (34), le joint conique (62) ayant sa dimension radiale maximale adjacente à la paroi (16) et sa dimension radiale minimale adjacente au moyeu (16); une autre face du joint conique (61) et l'une des faces extérieures du moyeu (34) formant une face extérieure conique essentiellement continue de façon à ce que l'interface (70) étanche soit située dans une zone à fort écoulement de fluide à l'intérieur du carter (12) et de façon à ce que l'air enformé à l'intérieur du fluide biologique ne s'accumule pas dans une zone adjacente à l'interface (70) étanche; et le moyeu (34) ayant une formé généralement conique dont le diamêtre maximum est adjacent au joint conique (62).

2. Une pompe centrifuge telle que revendiquée dans la revendication n° 1 caractérisé par le fait que l'interface (70) étanche est généralement transversale par rapport au sens axial.

3. Une pompe centrifuge telle que revendiquée dans la revendication n° 2 caractérisé par la fait que le joint conique comprend un stator (64) raccordé à la paroi (16) et entourant l'arbre (32), le stator (64) définissant une première face (74) du joint généralement transversale par rapport au sens axial et située en position intermédiaire entre la paroi (16) et le moyeu (34); un rotor (66) situé coaxialement sur l'arbre (32) entre le stator (64) et le moyeu (34), le rotor (66) définissant une seconde face (76) du joint faisant face à la première face (74) et généralement transversale par rapport au sens axial, le rotor (66) étant monté de façon à tourner avec la roue mobile (30) et pouvant se déplacer dans le sens axial; et une bague (68) élastique, placée coaxialement sur l'arbre (32), destinée à faire contacter les faces étanches une et deux (74, 76) de façon à ce qu'elles constituent l'interface (70) étanche aux fluides autour de l'arbre (32).

4. Une pompe centrifuge telle que revendiquée dans la revendication n° 3 caractérisé par la fait que le stator (64) est muni à son extrémité le plus proche de la paroi (16) d'une collerette (64A) se prolongeant radialement vers l'extérieur.

5. Une pompe centrifuge telle que revendiquée dans la revendication n° 3 caractérisé par la fait que le stator (64) est fabriqué à partir d'un métal; le rotor (55) est fabriqué à partir d'une bague en polymère basse friction; et le bague élastique (68) est fabriquée à partir d'une rondelle-ressort en élastomère.

6. Une pompe centrifuge telle que revendiquée dans la revendication n° 5 caractérisé par le fait que le stator (64) est fabriqué en aluminium; le polymère basse friction est une matière nylon; et l'élastomère est un caoutchouc silicone.

7. Une pompe centrifuge telle que revendiquée dans la revendication n° 3 caractérisé par le fait que la bague élastique (68) est située entre le moyeu (34) et le rotor (66) afin de pousser le rotor étanche (66) vers le stator (64).

8. Une pompe centrifuge telle que revendiquée dans la revendication n° 7, caractérisée par le fait que le rotor (66) comprend une rainure circulaire (92) dans sa face située face à la bague élastique (68), et que la bague élastique (68) porte une nervure circulaire (90) qui s'engage dans la rainure circulaire (92) du rotor (66).

9. Une pompe centrifuge telle que revendiquée dans la revendication n° 7 caractérisée par le fait que le moyeu (34) comprend une rainure circulaire (96) dans sa face (88) située face à la bague élastique (68), et que le bague élastique (68) porte une nervure circulaire (94) qui s'engage dans la rainure circulaire (96) du moyeu (34).

10. Une pompe centrifuge telle que revendiquée par l'une quelconque des revendications n° 1 à 9, caractérisée par le fait que l'arbre (32) est un arbre d'entraînement qui se prolonge en traversant la paroi (16) et dont une première extrémité (32A) se trouve dans la chambre d'aspiration (20) et une seconde extrémité se trouve sur un côté de la paroi (16) faisant face à la chambre d'aspiration (20); et par le fait que le moyeu (34) est fixé à la première extrémité (32A) de l'arbre d'entraînement.

11. Une pompe centrifuge telle que revendiquée par l'une quelconque des revendications n° 1 à 10, caractérisée par le fait que la roue mobile (30) est équipée d'un ensemble de premières aubes (36) fixées au moyeu (34) et se prolongeant à partir de ce moyeu (34), chacune de ces premières aubes (26) ayant un bord arrière (36A) généralement parallèle à la paroi (16) et positonné plus près de la paroi (16) que le moyeu (34), et chacune ayant un bord intérieur (36B) partant du bord arrière du moyeu (34) et étant généralement parallèle et à proche distance d'une face extérieure du joint conique (62).

12. Une pompe centrifuge telle que revendiquée dans la revendication n° 11 caractérisée par le fait que les bords arrières (36A) des premières aubes (36) sont à plus proche distance de la paroi (16) que l'interface étanche (70).

13. Une pompe centrifuge telle que revendiquée dans la revendication n° 11 caractérisée par le fait que la roue mobile (30) comprned en outre une couronne porte-aubes (40) maintenu par l'ensemble des premières aubes (36) en position radiale à partir du moyeu (34) et du joint conique (62), ainsi qu'un ensemble de secondes aubes plus courtes (38) maintenues par la couronne porte-aubes (40) à des emplacements situées entre les premières aubes (36), ces secondes aubes (38) étant positionnées radialement par rapport au moyeu (34).

14. Une pompe centrifuge telle que revendiquée dans la revendication n° 13 caractérisée par le fait que la couronne porte-aubes (40) a un bord intérieur qui est positionné radialement par rapport au moyeu (34), et que les premières et les secondes aubes (36, 38) maintenues par la couronne porte-aubes (40) se prolongent et dépassent le bord extérieur de la couronne porte-aubes (40).

Fig. 1

Fig. 2

Fig. 3

Fig. 4

Fig. 7

Fig. 5

Fig. 8

Fig. 6